Europäisches Patentamt

**European Patent Office**

**Office européen des brevets**

(19)

(11) Numéro de publication: **0 136 352**
**B1**

(12) # FASCICULE DE BREVET EUROPÉEN

(45) Date de publication du fascicule du brevet:
**22.07.87**

(51) Int. Cl.⁴: **A 61 K 7/48**

(21) Numéro de dépôt: **84901361.0**

(22) Date de dépôt: **23.03.84**

(86) Numéro de dépôt international:
**PCT/FR 84/00078**

(87) Numéro de publication internationale:
**WO 84/03835 (11.10.84 Gazette 84/24)**

(54) **COMPOSITION COSMETIQUE ET SON PROCEDE D'OBTENTION.**

(30) Priorité: **30.03.83 FR 8305283**

(43) Date de publication de la demande:
**10.04.85 Bulletin 85/15**

(45) Mention de la délivrance du brevet:
**22.07.87 Bulletin 87/30**

(84) Etats contractants désignés:
**AT BE CH DE GB LI LU NL SE**

(56) Documents cité:
**FR-A-1 278 065**
**FR-A-1 603 826**
**FR-A-2 121 192**
**FR-A-2 205 304**

**H. JANISTYN, "Handbuch der Kosmetika", Dr.**
**Alfred HÜTHIG Verlag, Heidelberg, 1978, page 233**

(73) Titulaire: **DURAFFOURD, Alain, 11 bis, avenue Victor Hugo, F-75116 Paris (FR)**
Titulaire: **DURAFFOURD, Jean-Max, 11 bis, avenue Victor Hugo, F-75116 Paris (FR)**

(72) Inventeur: **DURAFFOURD, Alain, 11 bis, avenue Victor Hugo, F-75116 Paris (FR)**
Inventeur: **DURAFFOURD, Jean-Max, 11 bis, avenue Victor Hugo, F-75116 Paris (FR)**

(74) Mandataire: **Bouju, André, Cabinet Bouju 38 avenue de la Grande Armée, F-75017 Paris (FR)**

## Description

La présente invention concerne une composition pour la régénération des cellules et des tissus de la peau.

L'invention vise également le procédé pour préparer cette composition.

La peau, plus encore que les autres organes, conserve sa jeunesse en renouvelant ses cellules usées. L'épiderme est constitué de cellules mortes, anucléées, qui se kératinisent et sont éliminées. Elles doivent être remplacées par des cellules jeunes qui se forment constamment dans le derme.

Cette régénération cellulaire physiologique consomme un certain nombre de matériaux indispensables que l'organisme amène à pied d'oeuvre par la voie sanguine dont les capillaires assurent la liaison avec le derme.

Si l'organisme est fatigué ou vieilli, les déficiences ralentissent ce renouvellement. Il en résulte un vieillissement de la peau, auquel on peut remédier en lui apportant directement les facteurs que l'organisme n'est plus en mesure de lui fournir de façon régulière et suffisante.

On connaît selon les FR-A-1 278 065, FR-A-1 603 826 et FR-A-2 205 304 diverses compositions cosmétiques renfermant des extraits végétaux qui contiennent des quantités infimes (non précisées) d'ADN.

Le but de la présente invention est de créer une composition permettant d'apporter à la peau les facteurs qui permettent d'éviter son vieillissement et qui assurent une régénération des cellules de la peau.

Suivant l'invention, cette composition est caractérisée en ce qu'elle renferme un macérat aqueux d'embryons de blé et/ou de soja, tamponné à pH sensiblement égal à 7 et additionné d'un anti-oxydant, ce macérat étant enrichi en ADN pur lui-même extrait d'embryons de blé et/ou de soja.

Cette composition apporte à la peau de l'ADN qui est un facteur essentiel de la multiplication cellulaire.

Cette composition présente par ailleurs les deux propriétés majeures suivantes:
- la tolérance et la bonne assimilation par la peau ; ainsi elle ne contient pas de produits animaux susceptibles d'incompatibilités;
- l'absence de facteurs cancérigènes, que l'ADN pourrait autrement porter au coeur des noyaux des cellules du derme récepteur.

L'application sur la peau de la composition conforme à l'invention permet de réactiver la multiplication cellulaire déficiente, en favorisant l'hydratation de la peau et l'assimilation des protéines. Cette composition stimule également la circulation dermique, donc la nutrition.

La composition conforme à l'invention renferme de préférence entre 1 et 10 % en poids environ d'ADN pur.

Selon un autre aspect de l'invention, le procédé pour préparer la composition conforme à l'invention, pour régénérer les cellules et les tissus de la peau, comprend les étapes suivantes:
- on broie des embryons de blé et/ou de soja en présence d'une solution d'extraction tamponnée à pH sensiblement égal à 9,5 renfermant du saccharose, de l'EDTA (éthylène diamine tétracétate) et du chlorure de sodium, on filtre et on remet en suspension le résidu dans une solution tris salin tamponnée à pH sensiblement égal à 7,4 additionnée de détergent anionique, après agitation à la température ordinaire, on ajoute du perchlorate de sodium, et on poursuit l'agitation à 0°C; on ajoute ensuite du chloroforme et de l'octanol puis on centrifuge la solution, on récupère la phase aqueuse renfermant l'ADN et on précipite l'ADN par addition d'éthanol glacé.

On ajoute l'ADN ainsi préparé à un macérat aqueux de germes de blé et/ou de soja, tamponné à pH sensiblement égal à 7 et additionné d'un anti-oxydant.

Pour vérifier si l'ADN est exempt de facteurs oncogènes, on le soumet au test des cristallisations sensibles et à l'action sur des cultures de cellules.

Si la vérification est positive, on redissout l'ADN dans une solution de chlorure de sodium et de citrate tamponnée à pH 7, puis on fait agir à environ 37°C la RNASE préalablement débarrassée de DNASE par chauffage à 80°C environ puis refroidissement jusqu'à 0°C environ, on fait agir ensuite la PRONASE à environ 37°C, puis on ajoute du chloroforme et de l'octanol et on précipite l'ADN avec de l'éthanol glacé.

On expose ci-après à titre d'exemple non limitatif, le mode opératoire de la préparation d'une composition selon l'invention.

100 grammes de germes de blé frais et 100 grammes de germes de soja frais sont mis à macérer, après broyage et addition de 20 grammes de levure de bière, dans un litre d'eau, auxquels on ajoute 200 grammes de glycérine, un tampon borate, pour maintenir le pH à 7 et un anti-oxydant. On obtient une solution A.

Parallèlement, 40 grammes d'embryons de blé et de soja sont broyés dans un mortier avec du sable de Fontainebleau en présence d'un tampon d'extraction (0,25 M de saccharose, 0,05 M d'EDTA, 0,05 M de NaCl à pH égal à 9,5). Le broyat obtenu est ensuite filtré sur 4 couches de gaze et le filtrat est centrifugé pendant 10 minutes à + 4°C à 25 000 g.

Le résidu est remis en suspension dans un bécher contenant 4 ml de tampon tris salin (0,15 M de NaCl 0,015 M de tris à pH égal à 7,4) auxquels on ajoute 1,2 ml de détergent anionique à 30 %. Après 15 minutes d'agitation à la température ordinaire, on ajoute 1,2 ml de perchlorate de sodium (NaCl 0,5M) et on poursuit l'agitation à 0°C. On ajoute alors 6 ml de chloroforme et 0,6 ml de n-octanol et on agite encore 15 minutes. La solution obtenue est centrifugée à + 4°C pendant 10 minutes à 25 000 g.

On obtient ainsi trois phases: une phase inférieure chloroformique, une croute intermédiaire protéique et une phase supérieure aqueuse contenant les acides nucléiques dont l'ADN. Cette phase est récupérée avec précaution. L'addition de deux volumes d'éthanol glacé précipite les fibres d'ADN. Ces fibres sont enroulées autour d'une baguette de verre et conservées au froid dans de l'acétone.

Les fibres ainsi obtenues contiennent essentiellement de l'ADN avec une petite quantité d'ARN et d'acides aminés. Il faut s'assurer que ces fibres n'emprisonnent pas de facteurs oncogènes ou cancérigènes. A cet effet on utilise la technique des cristallisations sensibles et l'action de ces fibres d'ADN sur des cultures de cellules. Si cette vérification est négative, on ajoute l'ADN à l'extrait aqueux précédemment préparé. Si la vérification est positive, il faut poursuivre la purification.

Après avoir enlevé de l'acétone et séché dans un courant d'air les fibres d'ADN on les redissout dans un minimum de solution de chlorure de sodium et de citrate (0,15 M NaCl, 0,015 M de citrate à pH 7).

Pour abaisser le taux de contaminants protéiques et ribonucléiques, on fait agir: la RNASE (concentration: 100 microgrammes par ml final dans 0,15 M de NaCl à pH 5) pendant 30 minutes à 37°C dans un bac thermostaté.

La RNASE utilisée à cet effet est préablement débarassée de toute DNASE contaminante par chauffage à 80°C pendant 10 minutes et refroidissement brusque à 0°C.

On fait agir ensuite la PRONASE pendant plusieurs heures à 37°C. La PRONASE doit être auparavant autodigérée pendant 2 heures à 37°C puis pendant 1 heure à 37°C sous agitation.

A la suite de l'action des enzymes RNASE et PRONASE, il est procédé à une dernière déprotéinisation de l'ADN au moyen d'un mélange de chloroforme et d'octanol, puis, après précipitation à l'éthanol glacé comme décrit précédemment, on enroule les fibres d'ADN purifié sur une baguette de verre.

On procéde enfin à un nouveau contrôle pour vérifier l'absence de facteurs oncogènes. On peut alors ajouter l'ADN à la solution A.

La composition préparée selon l'exemple que l'on vient de décrire renferme environ 5 % d'ADN pur et exempt de facteurs oncogènes, des vitamines A, B1, B2, B5, B12, E, K, D, H et PP, des oligo-éléments telsque du calcium, du fer, du manganése, du sodium, du potassium, du chlore, du soufre, du silicium, du zinc, du manganèse, du cobalt, du cuivre et de l'arsenic, et des acides aminés en petite quantité.

Cette composition est destinée à être commercialisée telle quelle ou sous forme diluée de façon à renfermer au moins 1 % d'ADN pur.

L'activité de l'ADN est de préférence renforcée en soumettant la solution à un courant d'ions négatifs.

La composition conforme à l'invention peut être mélangée à d'autres ingrédients cosmétiques ou pharmaceutiques pour permettre son application sur la peau, sous forme de lotion, crème, pommade et analogue.

On donne ci-après quelques exemples de formulations cosmétiques.

1) <u>lotion</u>:
Composition d'ADN : 10 grammes
tampon borate à pH 5,8: 250 ml
ricinoléate de sodium: 10 ml
eau distillée d'Hamamélis qsp: 1000 ml
parfum hypoallergique: qs

2) <u>crème</u>:
Composition d'ADN: 2,5 grammes
tampon borate à pH 5,8: 50 ml
huile d'amande: 20 ml
perhydrosqualène: 3 ml
émulsifiant: 20 grammes
eau distillée de rose qsp: 250 grammes
parfum hypoallergique: qs

La composition conforme à l'invention étant donné l'absence de toxicité de l'ADN peut également être présentée sous forme d'ampoules buvables ou injectables. A titre d'exemple la formulation d'une telle ampoule buvable ou injectable peut être la suivante:

ADN pur: 2,5 grammes
Sérum physiologique qsp: 10 ml

Les essais cliniques exposés ci-après montrent l'efficacité de la composition conforme à l'invention.

Il a été procédé à une série d'essais cliniques sur des personnes dont la peau ou les cheveux étaient susceptibles de bénéficier d'un traitement régénérateur effectif. L'association de l'ADN, des vitamines et des oligo éléments crée une véritable synergie régénératrice. Il a pu être relevé des résultats très positifs et souvent spectaculaires dans les cas suivants:

- deux cas de couperose,
- quatre cas de rides profondes au visage et au cou,
- deux cas d'hypovitalité de la peau sur un état général déficient,
- un cas de séborrhée,
- et trois cas de cellulite localisée.

Sur les cheveux il a été obtenu la disparition des pellicules, de la séborrhée, un arrêt de la chute et une régénération de cheveux anterieurement ternes et cassants.

Il a été constaté que la composition conforme à l'invention agit en réactivant la multiplication cellulaire déficiente, en favorisant l'hydratation de la peau et l'assimilation des protéines. De plus, il a été constaté que cette composition stimulait également la circulation dermique, donc la nutrition des cellules.

## Revendications

pour les Etats contractants: BE, CH, DE, GB, LI, LU, NL, SE.

1. Composition pour la régénération des cellules et des tissus de la peau, caractérisée en ce qu'elle renferme un macérat aqueux d'embryons de blé et/ou de soja, tamponné à pH sensiblement égal à 7 et additionné d'un anti-oxydant, ce macérat étant enrichi en ADN pur, lui-même extrait d'embryons de germes de blé et/ou de soja.

2. Composition conforme à la revendication 1, caractérisé en ce qu'elle renferme entre 1 et 10 % en poids d'ADN pur.

3. Composition conforme à l'une quelconque des revendications 1 ou 2, caractérisée en ce qu'elle renferme en outre des vitamines, des oligo-éléments et des acides aminés.

4. Procédé pour préparer une composition pour la régénération des cellules et des tissus de la peau, conforme à l'une des revendications 1 à 3, caractérisé par les étapes suivantes:

- on broie des embryons de blé et de soja en présence d'une solution d'extraction tamponnée à pH sensiblement égal à 9,5 renfermant du saccharose, de l'EDTA (éthylène diamine tétracétate) et du chlorure de sodium,

- on filtre et on remet en suspension le résidu dans une solution tris salin tamponnée à pH sensiblement égal à 7,4 additionnée de détergent anionique,

- après agitation à température ordinaire on ajoute du perchlorate de sodium et on poursuit l'agitation à 0°C,

- on ajoute ensuite du chloroforme et de l'octanol puis on centrifuge la solution, on récupère la phase aqueuse renfermant l'ADN, on précipite l'ADN par addition d'éthanol glacé et,

- on ajoute l'ADN ainsi préparé à un macérat aqueux de germes de blés et/ou de soja, tamponné à pH sensiblement égal à 7 et additionné d'un anti-oxydant.

5. Procédé conforme à la revendication 4, caractérisé en ce qu'on vérifie si l'ADN obtenu est exempt des facteurs oncogènes en le soumettant au test des cristallisations sensibles et à l'action sur des cultures de cellules et en ce que si la vérification est positive, on redissout l'ADN dans une solution de chlorure de sodium et de citrate tamponné à pH 7, puis on fait agir à environ 37°C la RNASE préalablement débarrassée de DNASE par chauffage à environ 80°C puis refroidissement brusque à 0°C; on fait agir ensuite la PRONASE à environ 37°C puis on ajoute du chloroforme et de l'octanol et on précipite l'ADN à l'éthanol glacé.

## Revendications

pour l'Etat contractant: AT.

1. Procédé pour préparer une composition pour la régénération des cellules et des tissus de la peau, caractérisé par les étapes suivantes:

- on broie des embryons de blé et de soja en présence d'une solution d'extraction tamponnée à pH sensiblement égal à 9,5 renfermant du saccharose, de l'EDTA (éthylène diamine tétracétate) et du chlorure de sodium,

- on filtre et on remet en suspension le résidu dans une solution tris salin tamponnée à pH sensiblement égal à 7,4 additionnée de détergent anionique,

- après agitation à température ordinaire on ajoute du perchlorate de sodium et on poursuit l'agitation à 0°C,

- on ajoute ensuite du chloroforme et de l'octanol puis on centrifuge la solution, on récupère la phase aqueuse renfermant l'ADN, on précipite l'ADN par addition d'éthanol glacé et,

- on ajoute l'ADN ainsi préparé à un macérat aqueux de germes de blé et/ou de soja, tamponné à pH sensiblement égal à 7 et additionné d'un anti-oxydant.

2. Procédé conforme à la revendication 1, caractérisé en ce qu'on vérifie si l'ADN obtenu est exempt des facteurs oncogènes en le soumettant au test des cristallisations sensibles et à l'action sur des cultures de cellules et en ce que si la vérification est positive, on redissout l'ADN dans une solution de chlorure de sodium et de citrate tamponné à'pH 7, puis on fait agir à environ 37°C la RNASE préalablement débarrassée de DNASE par chauffage à environ 80°C puis refroidissement brusque à 0°C; on fait agir ensuite la PRONASE à environ 37°C puis on ajoute du chloroforme et de l'octanol et on précipite l'ADN à l'éthanol glacé.

## Patentansprüche

für die Vertragsstaaten BE, CH, DE, GB, LI, LU, NL, SE.

1. Zusammensetzung für die Erneuerung der Zellen und Gewebe der Haut, dadurch gekennzeichnet, daß sie ein wäßriges Mazerat von Weizenkeimen und/oder Sojakeimen enthält, das genau auf pH 7 gepuffert und dem ein Antioxidationsmittel zugegeben ist, wobei das Mazerat an reiner DNS angereichert ist, welche selbst aus Weizenkeimen und/oder Sojakeimen extrahiert ist.

2. Zusammensetzung nach Anspruch 1, dadurch gekennzeichnet, daß sie zwischen 1 und 10 Gew.% reine DNS enthält.

3. Zusammensetzung nach einem der Ansprüche 1 oder 2, dadurch gekennzeichnet, daß sie zusätzlich Vitamine, Spurenelemente und Aminosäuren enthält.

4. Verfahren zur Herstellung einer Zusammensetzung für die Erneuerung der Zellen und der Gewebe der Haut gemäß einem der Ansprüche 1 bis 3, gekennzeichnet durch die folgenden Stufen:

- man zerstößt Weizen- und Sojakeime in Gegenwart einer genau auf pH 9,5 gepufferten Extraktionslösung, welche Saccharose, EDTA (Äthylendiamin-tetraacetat) und Natriumchlorid enthält,

- man filtriert und resuspendiert den Rückstand in einer kochsalzhaltigen Tris-Lösung, die genau auf pH 7,4 gepuffert ist und ein anionisches oberflächenaktives Mittel enthält,

- nach Rühren bei Raumtemperatur gibt man Natriumperchlorat zu und rührt weiter bei 0°C,
- man gibt anschließend Chloroform und Octanol zu, zentrifugiert dann die Lösung, trennt die wäßrige, DNS enthaltende Phase ab und fällt die DNS durch Zugabe von eiskaltem Äthanol aus,
- schließlich fügt man die so gewonnene DNS einem wäßrigen Mazerat von Weizenkeimen und/oder Sojakeimen zu, welches genau auf pH 7 gepuffert ist und ein Antioxidationsmittel enthält.

5. Verfahren nach Anspruch 4, dadurch gekennzeichnet, daß man prüft, ob die DNS frei von onkogenen Faktoren ist, indem man sie einem Test empfindlicher Kristallisationen und der Einwirkung auf Zellkulturen unterwirft, sowie dadurch, daß man, wenn die Prüfung positiv ausfällt, die DNS in einer auf pH 7 gepufferten Natriumchlorid- und Citrat-Lösung wieder auflöst, dann bei etwa 37°C die zuvor von DNASE durch Erhitzen auf etwa 80°C und anschließende Abschreckung auf 0°C befreite RNASE einwirken läßt und schließlich die PRONASE bei etwa 37°C einwirken läßt und dann Chloroform und Octanol hinzufügt und die DNS mit eiskaltem Äthanol ausfällt.

**Patentansprüche**

für den Vertragsstaat AT.

1. Verfahren zur Herstellung einer Zusammensetzung für die Erneuerung der Zellen und der Gewebe der Haut, gekennzeichnet durch die folgenden Stufen:
- man zerstößt Weizen- und Sojakeime in Gegenwart einer genau auf pH 9,5 gepufferten Extraktionslösung, welche Saccharose, EDTA (Äthylendiamin-tetraacetat) und Natriumchlorid enthält,
- man filtriert und resuspendiert den Rückstand in einer kochsalzhaltigen Tris-Lösung, die genau auf pH 7,4 gepuffert ist und ein anionisches oberflächenaktives Mittel enthält,
- nach Rühren bei Raumtemperatur gibt man Natriumperchlorat zu und rührt weiter bei 0°C,
- man gibt anschließend Chloroform und Octanol zu, zentrifugiert dann die Lösung, trennt die wäßrige, DNS enthaltende Phase ab und fällt die DNS durch Zugabe von eiskaltem Äthanol aus,
- schließlich fügt man die so gewonnene DNS einem wäßrigen Mazerat von Weizenkeimen und/oder Sojakeimen zu, welches genau auf pH 7 gepuffert ist und ein Antioxidationsmittel enthält.

2. Verfahren nach Anspruch 1, dadurch gekennzeichnet, daß man prüft, ob die DNS frei von onkogenen Faktoren ist, indem man sie einem Test empfindlicher Kristallisationen und der Einwirkung auf Zellkulturen unterwirft, sowie dadurch, daß man, wenn die Prüfung positiv ausfällt, die DNS in einer auf pH 7 gepufferten Natriumchlorid- und Citrat-Lösung wieder auflöst, dann bei etwa 37°C die zuvor von DNASE durch Erhitzen auf etwa 80°C und anschließende Abschreckung auf 0°C befreite RNASE einwirken läßt und schließlich die PRONASE bei etwa 37°C einwirken läßt und dann Chloroform und Octanol hinzufügt und die DNS mit eiskaltem Äthanol ausfällt.

**Claims**

for the Contracting states BE, CH, DE, GB, LI, LU, NL, SE.

1. A composition for the regeneration of the cells and tissues of the skin, characterised in that it contains an aqueous macerate of wheat and/or soybean embryos, buffered to a pH of substantially 7 and added with an anti-oxidant, said macerate being enriched in pure DNA extracted itself from wheat and or soybean germ embryos.

2. A composition according to claim 1, characterised in that it contains between 1 and 10 % by weight of pure DNA.

3. A composition according to claim 1 or 2, characterised in that it also contains vitamins, trace elements and amino acids.

4. A process for the preparation of a composition for the regeneration of the cells and tissues of the skin in accordance with any one of claims 1 to 3, characterised by the following steps:
- wheat and soybean embryos are crushed in the presence of an extraction solution buffered to a pH of substantially 9.5 containing saccharose, EDTA (ethylene diamine tetracetate) and sodium chloride,
- the solution is filtered and the residue suspended in a saline tris solution buffered to a pH of substantially 7.4 to which an anionic detergent has been added,
- after agitation at room temperature sodium perchlorate is added and agitation is continued at 0°C,
- chloroform and octanol are then added whereafter the solution is centrifuged, the DNA-containing aqueous phase is recovered and the DNA is precipitated by the addition of iced ethanol, and
- an aqueous macerate of wheat and/or soybean germs, buffered to a pH substantially 7 and added with an ainti-oxidant, is added to the so purified DNA.

5. A process according to claim 4, characterised in that a check is carried out as to whether the obtained DNA is free from oncogenous factors by subjecting it to the sensitive crystallization test and testing its action on cell cultures, and in that, if the check is positive, the DNA is redissolved in a solution of sodium chloride and citrate buffered to pH 7, whereafter RNASE, from which the DNASE has been previously removed by heating to about 80°C followed by rapid cooling to 0°C, is allowed to act at about 37°C; PRONASE is then allowed to act at about 37°C whereafter chloroform and octanol are added and the DNA is precipitated with iced ethanol.

## Claims

for the Contracting state AT.

1. A process for the preparation of a composition for the regeneration of the skin cells and tissues characterised by the following steps:

- wheat and soya embryos are crushed in the presence of an extraction solution buffered to a pH of substantially 9.5 containing saccharose, EDTA (ethylene diamine tetracetate) and sodium chloride,

- the solution is filtered and the residue suspended in a saline tris solution buffered to a pH of substantially 7.4 to which an anionic detergent has been added,

- after agitation at room temperature sodium perchlorate is added and agitation is continued at 0°C,

- chloroform and octanol are then added whereafter the solution is centrifuged, the DNA containing aqueous phase is recovered and the DNA is precipitated by the addition of iced ethanol, and,

- an aqueous macerate of wheat and/or soybean germs, buffered to a pH of substantially 7 and added with an anti-oxidant, is added to the so purified DNA.

2. A process according to claim 1, characterised in that a check is carried out as to whether the DNA is free from oncogenous factors by subjecting it to the sensitive crystallization test and testing its action on cell cultures, and in that, if the check is positive, the DNA is redissolved in a solution of sodium chloride and citrate buffered to pH 7, whereafter RNASE, from which the DNASE has been previously removed by heating to about 80°C followed by rapid cooling to 0°C, is allowed to act at about 37°C; PRONASE is then allowed to act at about 37°C whereafter chloroform and octanol are added and the DNA is precipitated with iced ethanol.